(19) Europäisches Patentamt
European Patent Office
Office européen des brevets

(11) EP 1 767 588 A1

(12) **EUROPEAN PATENT APPLICATION**
published in accordance with Art. 158(3) EPC

(43) Date of publication:
**28.03.2007 Bulletin 2007/13**

(21) Application number: **05734511.8**

(22) Date of filing: **13.04.2005**

(51) Int Cl.:
***C09B 57/10*** *(2006.01)* ***C07D 213/79*** *(2006.01)*
***H01L 31/04*** *(2006.01)* ***H01M 14/00*** *(2006.01)*
*C07F 15/00* *(2006.01)* *C09K 3/00* *(2006.01)*

(86) International application number:
**PCT/JP2005/007486**

(87) International publication number:
**WO 2005/100484 (27.10.2005 Gazette 2005/43)**

(84) Designated Contracting States:
**DE GB**

(30) Priority: **16.04.2004 JP 2004121963**
**20.04.2004 JP 2004123619**

(71) Applicant: **JSR Corporation**
**Tokyo 104-0045 (JP)**

(72) Inventors:
- **WANG, Yong**
  **c/o JSR Corporation**
  **Chuo-ku, Tokyo 104-0045 (JP)**
- **MATSUKI, Yasuo**
  **c/o JSR Corporation**
  **Chuo-ku, Tokyo 104-0045 (JP)**

(74) Representative: **TBK-Patent**
**Bavariaring 4-6**
**80336 München (DE)**

(54) **DYE AND DYE-SENSITIZED SOLAR CELL**

(57) A novel dye which has high conversion efficiency, excellent weatherability and heat resistance when it is used in a dye-sensitized solar cell and a dye-sensitized solar cell comprising this dye.

This dye is represented by the following formula (1):

$$ML^1L^2X^1X^2 \qquad (1)$$

wherein M is an element of any one of the groups 8 to 10 of the long form of the periodic table, $L^1$ and $L^2$ are each independently a bidentate ligand composed of a specific bipyridine, and $X^1$ and $X^2$ are each independently a monovalent atomic group or unidentate ligand.

EP 1 767 588 A1

## Description

TECHNICAL FIELD

The present invention relates to a dye and a dye-sensitized solar cell comprising the same.

BACKGROUND ART

Along with growing interest in energy problems, researches into solar cells capable of converting light, particularly sunlight, into electricity efficiently are now under way. Silicon-based solar cells making use of amorphous silicon or polycrystal silicon are becoming popular. However, the silicon-based solar cells are expensive and have a problem with the supply of high-purity silicon, and it is said that the spread of the silicon-based solar cells is limited.

Dye-sensitized solar cells are now attracting much attention. The dye-sensitized solar cells are expected to have a large number of advantages as compared with silicon-based solar cells. For example, the solar-to-electric energy conversion efficiency of the solar cell is high, the solar cell can be manufactured at a low cost, an inexpensive oxide semiconductor such as titanium oxide can be used as a raw material without being purified at a high level, and equipment used for the manufacture of the solar cell is inexpensive (refer to US Patent No. 4927721 and WO98/50393).

It is known that the solar-to-electric energy conversion efficiency, weatherability and heat resistance of a dye-sensitized solar cell greatly depend upon the dye used.

A dye called "N719" represented by the following formula (4) and a dye called "black dye" represented by the following formula (5) are widely used as conventionally known dyes (refer to J. Am. Chem. Soc., 115, 6382-6390 (1993) and J. Am. Chem. Soc., 123, 1613-1624 (2001)).

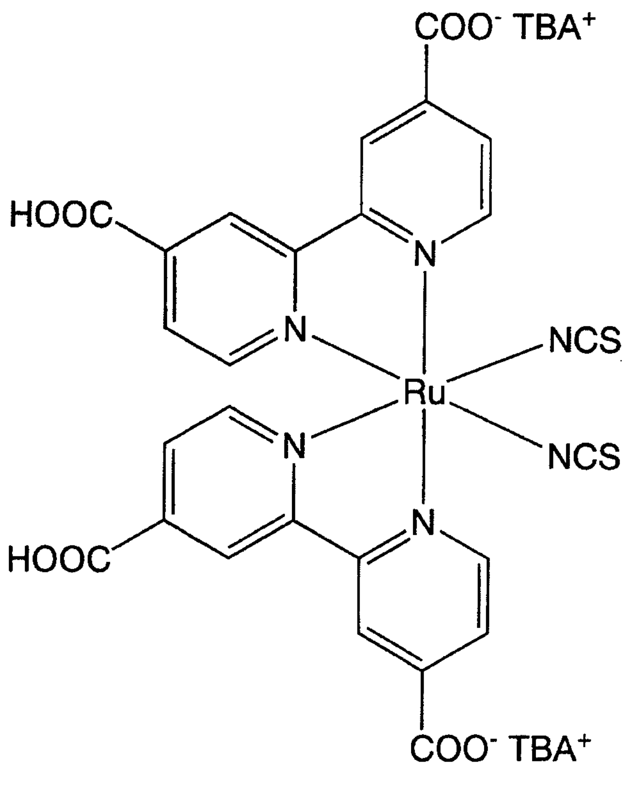

... (4)

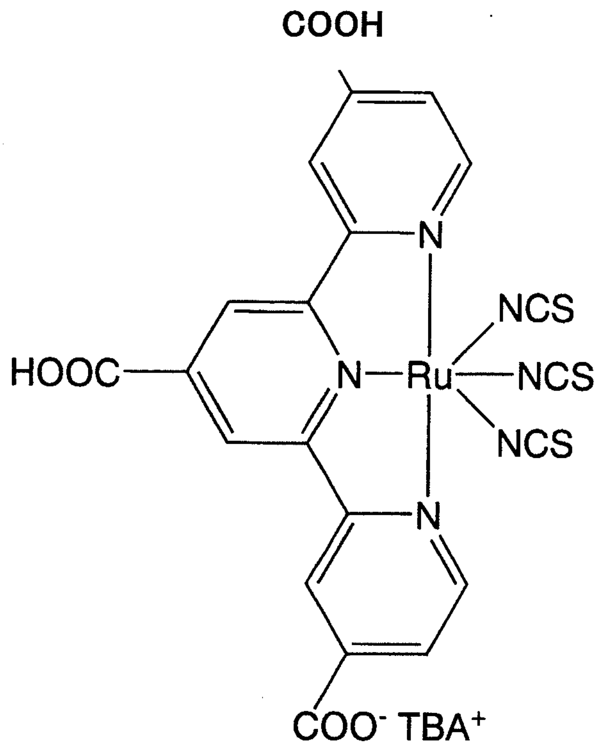

... (5)

In the formulas (4) and (5), $TBA^+$ represents a tetrabutyl ammonium ion.

However, these dyes are excellent in terms of quantum yield but unsatisfactory in terms of conversion efficiency, weatherability and heat resistance as a solar cell. The development of a more excellent dye is awaited.

DISCLOSURE OF THE INVENTION

It is an object of the present invention which has been made in view of the above situation to provide a novel dye which exhibits high conversion efficiency, excellent weatherabilty and heat resistance when it is used in a dye-sensitized solar cell and a dye-sensitized solar cell comprising the same.

Other objects and advantages of the present invention will become apparent from the following description.

According to the present invention, firstly the above objects and advantages of the present invention are attained by a dye represented by the following formula (1).

$$ML^1L^2X^1X^2 \qquad (1)$$

In the above formula (1), M is an element of any one of the groups 8 to 10 of the long form of the periodic table, $L^1$ and $L^2$ are each independently either one of bidentate ligands represented by the following formulas (2) and (3), and $X^1$ and $X^2$ are each independently a monovalent atomic group or unidentate ligand.

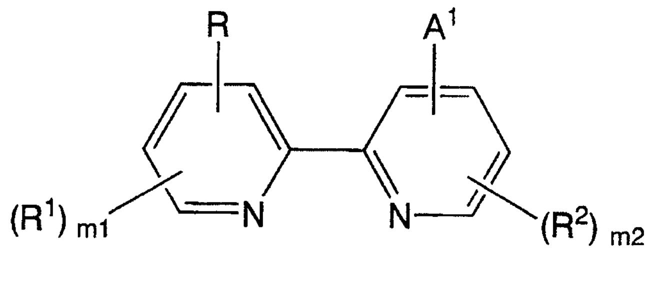

... (2)

In the above formula (2), $A^1$ is a carboxyl group, sulfonic acid group, phosphoric acid group or group corresponding to a salt thereof, R, $R^1$ and $R^2$ are each independently a monovalent organic group, and m1 and m2 are each independently an integer of 0 to 3.

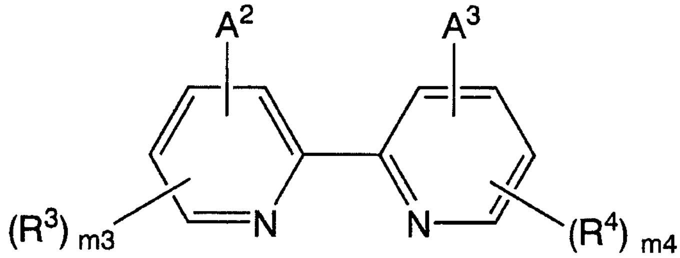

... (3)

In the above formula (3), $A^2$ and $A^3$ are each independently a carboxyl group, sulfonic acid group, phosphoric acid group or group corresponding to a salt thereof, $R^3$ and $R^4$ are each independently a monovalent organic group, and m3 and m4 are each independently an integer of 0 to 3. When both $L^1$ and $L^2$ are bidentate ligands represented by the formula (3), both $A^2$ and $A^3$ are not carboxyl groups or groups corresponding to a salt thereof.

According to the present invention, secondly, the above objects and advantages of the present invention are attained by a dye-sensitized solar cell comprising the above dye.

PREFERABLE EMBODIMENT OF THE INVENTION

The dye of the present invention is represented by the above formula (1). In the formula (1), M is an element of any one of the groups 8 to 10 of the long form of the periodic table, $L^1$ and $L^2$ are each independently either one of bidentate ligands represented by the above formulas (2) and (3), and $X^1$ and $X^2$ are each independently a monovalent atomic group or unidentate ligand.

In the above formula (2), $A^1$ is a carboxyl group, sulfonic acid group, phosphoric acid group or group corresponding to a salt thereof, R, $R^1$ and $R^2$ are each independently a monovalent organic group, and m1 and m2 are each independently an integer of 0 to 3.

In the above formula (3), $A^2$ and $A^3$ are each independently a carboxyl group, sulfonic acid group, phosphoric acid group or group corresponding to a salt thereof, $R^3$ and $R^4$ are each independently a monovalent organic group, and m3 and m4 are each independently an integer of 0 to 3.

Examples of M include iron, ruthenium and osmium of the group 8, cobalt, rhodium and iridium of the group 9, and nickel, palladium and platinum of the group 10. Out of these, ruthenium is particularly preferred.

$A^1$ in the formula (2) and $A^2$ and $A^3$ in the formula (3) are each independently a carboxyl group, sulfonic acid group, phosphoric acid group or group corresponding to a salt thereof. Out of these, they are preferably a carboxyl group or a group corresponding to a salt thereof.

When $A^1$, $A^2$ or $A^3$ is a group corresponding to a salt, examples of the counter cation include ammonium ion, dimethylammonium ion, diethylammonium ion, tetramethylammonium ion, tetraethylammonium ion, tetrapropylammonium ion, tetrabutylammonium ion, sodium ion and potassium ion.

Preferred examples of the monovalent organic group represented by R in the formula (2) include organic groups represented by the following formulas (6) to (9).

$-R^5$ ... (6)

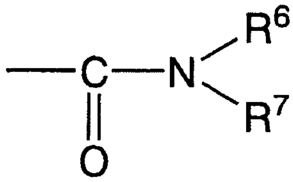

... (7)

$-O-R^8$ ... (8)

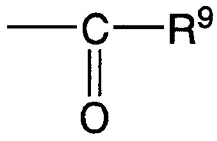 ... (9)

In the above formulas (6) to (9), $R^5$ to $R^9$ are each independently an alkyl group having 1 to 50 carbon atoms or a group represented by the following formula (10):

$$-(CH_2)_{m5}-(O-C_2H_4)_{m6}-O-R^{10} \quad \text{... (10)}$$

wherein $R^{10}$ is a hydrogen atom or alkyl group having 1 to 20 carbon atoms, m5 is an integer of 0 to 20, and m6 is an integer of 1 to 20.

When $R^5$ to $R^9$ are alkyl groups having 1 to 50 carbon atoms, the alkyl groups may be linear or branched.

The group represented by the above formula (7) is preferably an alkylaminocarbonyl group having 3 to 50 carbon atoms.

The monovalent organic group represented by $R^1$ and $R^2$ in the above formula (2) and $R^3$ and $R^4$ in the above formula (3) is, for example, an alkyl group having 1 to 4 carbon atoms or an alkoxy group.

Further, examples of the monovalent atomic group or unidentate ligand represented by $X^1$ and $X^2$ in the above formula (1) include atomic groups or ligands represented by the following formulas (11) to (17).

$^-N{=}C{=}S$ ... (11)

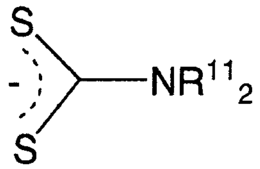 ... (12)

$^-N{\cdots}C{\cdots}NAr$ ... (13)

$Cl^-$ ... (14)

$^-N{\equiv}C$ ... (15)

$OH_2$ ... (16)

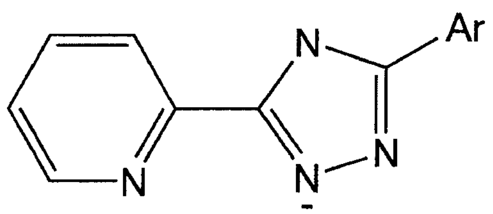 ... (17)

In the above formula (12), $R^{11}$ is an alkyl group having 1 to 6 carbon atoms. In the formulas (13) and (17), Ar is an aryl group having 6 to 12 carbon atoms.

Out of these, $X^1$ and $X^2$ are preferably an isothiocyanato represented by the above formula (11).

The dye of the present invention as above can be advantageously used in a dye-sensitized solar cell.

The dye-sensitized solar cell of the present invention comprising the dye of the present invention has at least a cathode, an anode opposed to the cathode, and an electrolyte held between the cathode and the anode. The cathode has an oxide thin film electrode which chemically adsorbs the dye of the present invention on transparent conductive glass. Tin oxide or indium-tin oxide (ITO) may be used as the transparent conductive glass.

Examples of the material forming the oxide thin film electrode include titanium oxide, niobium oxide, zinc oxide, tin oxide, tungsten oxide and indium oxide. Out of these, titanium oxide, niobium oxide and tin oxide are preferred and titanium oxide is particularly preferred. The method of forming an oxide thin film electrode is not particularly limited. For example, it can be manufactured advantageously by forming oxide fine particles which will become an oxide thin film electrode, suspending them in a suitable solvent, applying the resulting suspension to transparent conductive glass, removing the solvent and heating the coating film.

To adsorb the dye of the present invention to the oxide thin film electrode, a suitable method may be employed. For example, the dye of the present invention can be adsorbed to the oxide thin film electrode by immersing the above obtained transparent conductive glass having an oxide thin film electrode on the surface in a solution containing the dye of the present invention. Examples of the solvent which can be used herein include diethyl ether, acetonitrile and ethanol. The concentration of the dye in the solution is preferably 0.1 to 10 mmol/l. The immersion time is preferably 0.5 to 100 hours, more preferably 2 to 50 hours. The immersion temperature is preferably 0 to 100°C, more preferably 10 to 50°C.

The anode is not particularly limited if it has conductivity. For example, transparent conductive glass having a trace amount of platinum or conductive carbon deposited thereon can be preferably used.

A solution, solid or ionic liquid containing a redox system may be used as the electrolyte. An example of the electrolyte is an electrolyte solution containing a system making use of the following reaction of iodine as a redox system and acetonitrile or propionitrile as a solvent.

$$I_3^- + 2e^- = 3I^- + I_2$$

As described above, according to the present invention, there is provided a novel dye which exhibits high conversion efficiency, excellent weatherability and heat resistance when it is used in a dye-sensitized solar cell. The dye-sensitized solar cell of the present invention comprising the above dye has high conversion efficiency and excellent weatherability and heat resistance.

Examples

The following examples are provided to further illustrate the present invention.

Example 1

Synthesis of ligand

40 g of 4,4'-dimethyl-2,2'-bipyridine was added to 1 liter of concentrated sulfuric acid having a concentration of 98 wt% little by little under stirring to be dissolved in the concentrated sulfuric acid. 55 g of potassium dichromate was added to the resulting solution little by little while the temperature of the solution was maintained at 65°C or lower. The reaction mixture was left to be cooled to room temperature (23°C) and poured into 12 liters of iced water under stirring. After 2 hours of stirring, the precipitate was collected by filtration and rinsed with water. The obtained solid was re-dissolved in ether and the resulting solution was let pass through a silica gel column to be purified, and the solvent was removed to obtain 3.8 g of a product. It was found that the product was 4-carboxy-4'-methyl-2,2'-bipyridine by $^1$H-NMR analysis.

Synthesis of dye

0.3 g of (p-cymene) ruthenium (II) dichloride dimer was dissolved in 150 ml of N,N-dimethylformamide, and 0.205 g of 4-carboxy-4'-methyl-2,2'-bipyridine synthesized above was added to the resulting solution. After this mixture was stirred at 60°C in a nitrogen atmosphere for 4 hours, 0.234 g of 4,4'-dicarbonyl-2,2'-bipyridine was added and refluxed for 4 hours. Thereafter, 3.5 g of potassium isothiocyanate was added and refluxed for another 4 hours.

The reaction mixture was left to be cooled to room temperature (23°C), N,N-dimethylformamide was removed under reduced pressure, and 550 ml of water was added. After diluted nitric acid was added under stirring at room temperature to adjust the pH of the resulting solution to 2.5, the precipitate was collected by filtration. This solid was re-dissolved in methanol, and the resulting solution was let pass through the Sephadex LH-20 column (product commercially available from Amersham Biosciences Co., Ltd.) to be purified so as to obtain 0.12 g of a product. It was found that the product was represented by the following formula (18) by $^1$H-NMR analysis. This product was designated as "J1".

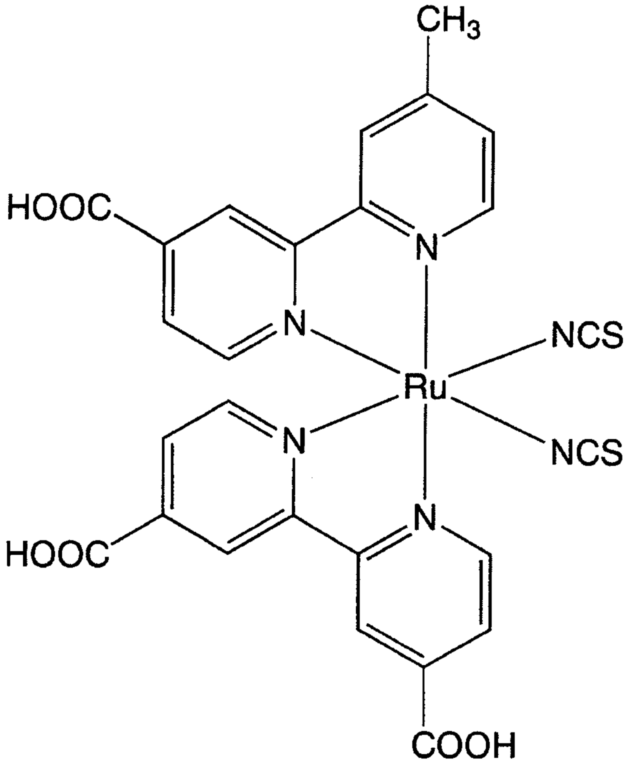

... (18)

Manufacture of dye-sensitized solar cell

12 g of titanium oxide fine particles and 0.2 g of the Triton X-100 dispersant (product commercially available from Aldrich Co., Ltd.) were added to a mixed solvent of 0.4 ml of acetylacetone and 20 ml of ion exchange water to prepare a dispersion. This dispersion was applied to a 1 mm-thick conductive glass substrate (ITO, resistance value = 10 $\Omega/cm^2$) and heated at 500°C in the air for 1 hour to obtain a conductive glass substrate having a titanium oxide thin film on the surface. This glass substrate was immersed in an ethanol solution containing the dye "J1" synthesized above in a concentration of 0.2 mmol/l at room temperature for 24 hours to manufacture a cathode having an oxide thin film electrode which chemically adsorbed the dye of the present invention on the transparent conductive glass.

Meanwhile, platinum was deposited on another conductive glass substrate (thickness of 1 mm, ITO, resistance value = 10 $\Omega/cm^2$) to manufacture an anode.

Further, an electrolyte solution containing 0.1 mol/l of iodine and 0.5 mol/l of lithium iodide dissolved in acetonitrile was prepared.

A dye-sensitized solar cell was manufactured by making the above cathode and anode opposed to each other and holding the above electrolyte solution between them.

Evaluation of dye-sensitized solar cell

When artificial sunlight was applied to the dye-sensitized solar cell manufactured as described above by using the WXS-50S-1.5 solar simulator (of WACOM Co., Ltd.) at an illuminance of 1,000 $W/m^2$ to measure the initial photoelectric conversion efficiency, it was 6.5 %. Thereafter, when the application of artificial sunlight was continued and the time elapsed until the photoelectric conversion efficiency became half of the initial value was measured as half-value period, it was 1,200 hours.

Example 2

Synthesis of ligand

100 ml of tetrahydrofuran was added to 11 ml of a tetrahydrofuran solution of lithium diisopropylamide having a concentration of 2 mol/l, and the resulting solution was cooled to -70°C. 2.0 g of powders of 4-carboxy-4'-methyl-2,2'-bipyridine synthesized in the step "synthesis of ligand" in Example 1 was added gradually to the solution under stirring

at -70°C. After 1 hour of agitation at -10°C, 100 ml of a tetrahydrofuran solution containing 5.5 g of dodecyl bromide was added dropwise to the resulting solution at -10°C. The reaction mixture was heated at room temperature and further kept stirred for another 1 hour.

Thereafter, 250 ml of iced water was added, and the pH of the resulting solution was adjusted to 2.0 with concentrated hydrochloric acid. The water layer was extracted with ether, concentrated, dried and let pass through a silica gel column to be purified, and the solvent was removed to obtain 1.1 g of a product. It was found that the product was 4-carboxy-4'-tridecyl-2,2'-bipyridine by $^{1}$H-NMR analysis.

Synthesis of dye

The procedure of Example 1 was substantially repeated except that 0.38 g of 4-carboxy-4'-tridecyl-2,2'-bipyridine synthesized above was used in place of 0.205 g of 4-carboxy-4'-methyl-2,2'-bipyridine in the "synthesis of dye" in Example 1 to obtain 0.18 g of a product. It was found that the product was represented by the following formula (19) by $^{1}$H-NMR analysis. This product was designated as "J2".

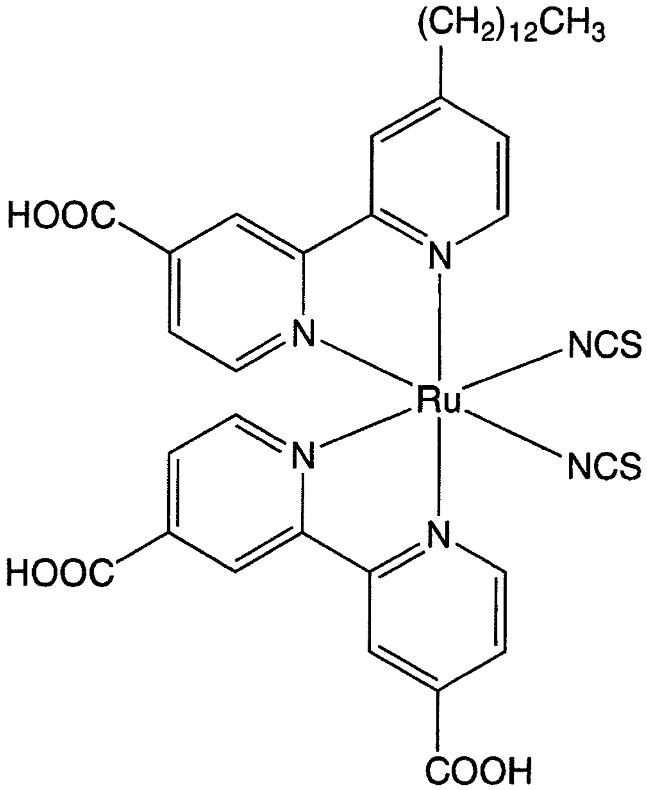

... (19)

Manufacture and evaluation of dye-sensitized solar cell

A dye-sensitized solar cell was manufactured substantially in the same manner as in Example 1 except that the dye "J2" was used in place of the dye "J1" used in the "manufacture of dye-sensitized solar cell" in Example 1 and evaluated in the same manner as the "evaluation of dye-sensitized solar cell" in Example 1. The results are shown in Table 1.

Example 3

Synthesis of ligand

30 g of 4,4'-dicarboxy-2,2'-bipyridine and 500 g of thionyl chloride were refluxed for 3 hours. After unreacted thionyl chloride was removed, 500 ml of methylene chloride, 17 g of diethylamine and 1.5 g of 4-dimethylaminopyridine were added and stirred at room temperature for 24 hours. Then, the reaction mixture was washed with diluted hydrochloric acid and purified by a silica gel column to remove the solvent so as to obtain 5.6 g of a product. It was found that the

product was 4-carboxy-4'-N,N-diethylaminocarbonyl-2,2'-bipyridine by $^1$H-NMR analysis.

Synthesis of dye

The procedure of Example 1 was substantially repeated except that 0.287 g of 4-carboxy-4'-N,N-diethylaminocarbonyl-2,2'-bipyridine synthesized above was used in place of 0.205 g of 4-carboxy-4'-methyl-2,2'-bipyridine used in the "synthesis of dye" in Example 1 to obtain 0.15 g of a product. It was found that the product was represented by the following formula (20) by $^1$H-NMR analysis. This product was designated as "J3".

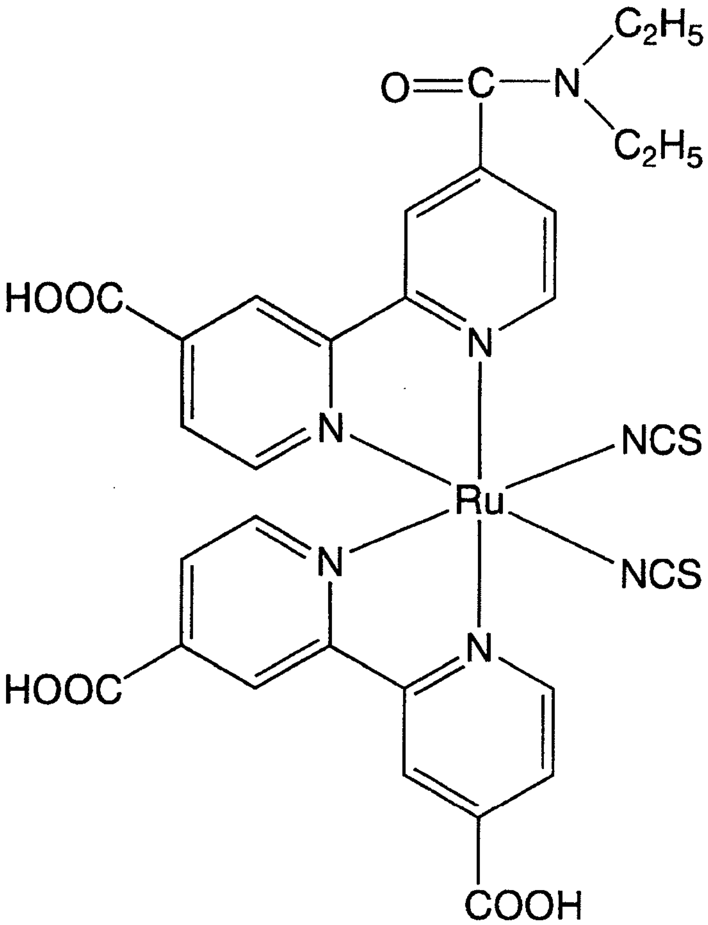

... (20)

Manufacture and evaluation of dye-sensitized solar cell

A dye-sensitized solar cell was manufactured substantially in the same manner as in Example 1 except that the dye "J3" was used in place of the dye "J1" used in the "manufacture of dye-sensitized solar cell" in Example 1 and evaluated in the same manner as the "evaluation of dye-sensitized solar cell" in Example 1. The results are shown in Table 1.

Example 4

Synthesis of ligand

The procedure of Example 3 was substantially repeated except that 46 g of N-methyl-N-dodecylamine was used in place of 17 g of diethylamine used in the "synthesis of ligand" in Example 3 to obtain 3.2 g of a product. It was found that the product was 4-carboxy-4'-N-methyl-N-dodecylaminocarbonyl-2,2'-bipyridine by $^1$H-NMR analysis.

Synthesis of dye

The procedure of Example 1 was substantially repeated except that 0.408 g of 4-carboxy-4'-N-methyl-N-dodecylaminocarbonyl-2,2' -bipyridine synthesized above was used in place of 0.205 g of 4-carboxy-4'-methyl-2,2'-bipyridine used in the "synthesis of dye" in Example 1 to obtain 0.21 g of a product. It was found that the product was represented by the following formula (21) by $^{1}$H-NMR analysis. This product was designated as "J4".

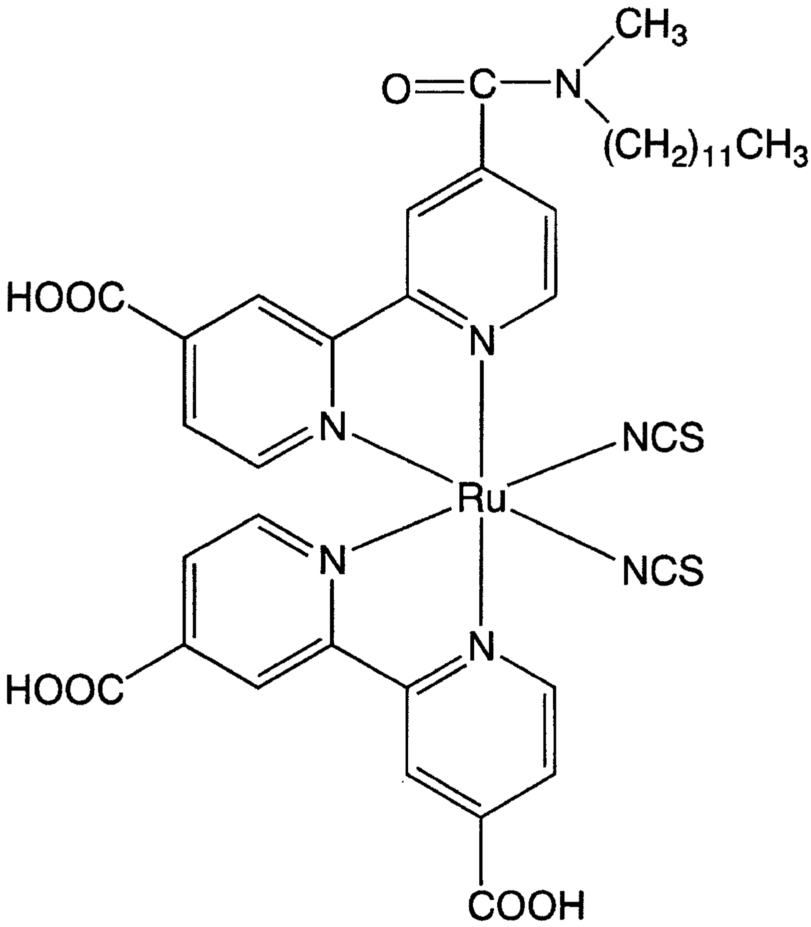

... (21)

Manufacture and evaluation of dye-sensitized solar cell

A dye-sensitized solar cell was manufactured substantially in the same manner as in Example 1 except that the dye "J4" was used in place of the dye "J1" used in the "manufacture of dye-sensitized solar cell" in Example 1 and evaluated in the same manner as the "evaluation of dye-sensitized solar cell" in Example 1. The results are shown in Table 1.

Comparative Example 1

Manufacture and evaluation of dye-sensitized solar cell

A dye-sensitized solar cell was manufactured substantially in the same manner as in Example 1 except that commercially available dye N3 (of Solaronix Co., Ltd., dye having a structure that two tetrabutylammonium ions are substituted by hydrogen ions in the structure represented by the above formula (4)) was used in place of the dye "J1" used in the "manufacture of dye-sensitized solar cell" in Example 1 and evaluated in the same manner as the "evaluation of dye-sensitized solar cell" in Example 1. The results are shown in Table 1.

Table 1

| | Dye | Photoelectric conversion efficiency (%) | Half-value period (hours) |
|---|---|---|---|
| Ex. 1 | J1 | 6.5 | 1200 |
| Ex. 2 | J2 | 7.2 | 1840 |
| Ex. 3 | J3 | 6.6 | 1130 |
| Ex. 4 | J4 | 7.1 | 1930 |
| C. Ex. 1 | N3 | 6.2 | 860 |
| Ex.: Example, C.Ex.: Comparative Example | | | |

Example 5

Synthesis of ligand

6.39 g of 4,4'-dimethyl-2,2'-bipyridine, 4.16 g of selenium dioxide and 375 ml (386.4 g) of 1,4-dioxane were charged into a vessel, refluxed for 24 hours and thermally filtered. After the filtrate was concentrated, 225 ml of ethanol and an aqueous solution of silver nitrate (6.48 g/50 ml) were added to the concentrated filtrate, and further 100 ml of an aqueous solution of sodium hydroxide having a concentration of 1.5 mol/l was added to the resulting solution. This solution was stirred at room temperature for 15 hours. The solution was then filtered. The ethanol in the filtrate was removed under reduced pressure. The residual solution was washed with 150 ml of chloroform. When a mixed solution of acetic acid and 4N hydrochloric acid in a volume ratio of 1:1 was added to the solution after washing to adjust the pH of the solution to 3.5, a white solid precipitated out. This solid was left to stand at 10°C for 24 hours, separated by filtration and dried. This solid was extracted with isopropyl alcohol, then the solvent was removed under reduced pressure to obtain 2.26 g of a product. It was found that the product was 4-carboxy-4'-methyl-2,2'-bipyridine by $^1$H-NMR analysis.

$^1$H-NMR (DMSO-d6,298K, 270MHz, δ(ppm)) ; δ = 8.86 (d, 1H), 8.82 (s, 1H), 8.58 (d, 1H), 8.27 (s, 1H), 7.86 (s, 1H), 7.33 (d, 1H), 2.44 (s, 3H, Me)

synthesis of dye

50 mg of (p-cymene) ruthenium(II) dichloride and 39.08 mg of 4,4'-dicarboxy-2,2'-bipyridine which had been dried under reduced pressure were added to 25 ml of anhydrous N,N-dimethylformamide which had been left in a nitrogen atmosphere after vacuum degasssing, and the resulting mixture was left in a nitrogen flow for 10 minutes. After this solution was stirred at 60°C for 4 hours in a nitrogen atmosphere, 34.28 mg of 4-carboxy-4'-methyl-2,2'-bipyridine (synthesized above) which had been dried under reduced pressure was added to the solution and left in a nitrogen flow for 10 minutes. Subsequently, this solution was stirred at 150°C in a nitrogen atmosphere for 4 hours and left to be cooled to 100°C, and 155.49 mg of potassium isothiocyanate dissolved in 2.5 ml of ion exchanged water was added to this solution and further stirred at 150°C for 4 hours. The reaction mixture was left to be cooled to room temperature, the solvent was removed under reduced pressure, and an aqueous solution containing 0.87 wt% of sodium carbonate was added. When 0.5 N nitric acid was added dropwise little by little to the resulting solution under stirring at room temperature to adjust its pH to 3. 0, a precipitate was obtained. This precipitate was left to stand for one night and centrifuged to collect a solid. The collected solid was washed with a small amount of ion exchanged water 3 times and freeze-dried. This solid was dissolved in a small amount of N,N-dimethylformamide and purified by column chromatography using the Sephadex LH-20 (product commercially available from Amersham Biosciences Co., Ltd.) to obtain 60 mg of a product. It was found that the product was represented by the above formula (18) by $^1$H-NMR analysis.

$^1$H-NMR (DMSO-d6, 298K, 270MHz, δ(ppm)) ; δ = 9.41 (m), [9.11-8.74 (m), 8.33 (m), 7.77-7.10 (m) ; COH], 2.42 (s, 3H)

Purification using the Sephadex LH-20 column was carried out by the following procedure.

The commercially available Sephadex LH-20 gel was immersed for one night to be swollen and charged into a column (3 x 60 cm). 400 ml of N,N-dimethylformamide was caused to flow into the column and then 300 ml of an N, N-dimethylformamide solution containing 0.1 wt% of lithium chloride was caused to flow into the column. The total amount of the crude dye product was dissolved in 5 ml of N,N-dimethylformamide and loaded into the column to elute a dye with an N,N-dimethylformamide solution containing 0.1 wt% of lithium chloride. The eluting dye was collected, the solvent was removed under reduced pressure, lithium chloride was removed by rinsing, and the dye was collected by centrifugation and dried under reduced pressure to obtain a purified dye.

Manufacture and evaluation of dye-sensitized solar cell

The Ti-Nanoxide D/SP titanium oxide paste of Solaronix Co., Ltd. (average particle diameter of titanium oxide of 13 nm) was applied to a 1.1 mm-thick conductive glass substrate (glass substrate having an ITO (indium-tin-oxide) thin film on the surface, resistivity of 10 $\Omega/cm^2$) and heated at 500°C in the air for 30 minutes. This treated glass substrate was immersed in an aqueous solution containing the dye obtained above in a concentration of 0.2 mmol/l at room temperature for 12 hours, left to stand at 23°C for 1 hour and dried to obtain a glass substrate having a titanium oxide layer to which the dye was adsorbed.

Separate from this, platinum was sputtered on a conductive glass substrate (resistivity of 10 $\Omega/cm^2$) to prepare an opposite electrode.

The two glass substrates manufactured above were opposed to each other with the titanium oxide layer and the platinum layer on the inner sides at an interval of 100 μm, and an acetonitrile solution containing 0.1 mol/l of iodine and 1.5 mol/l of lithium iodide was charged into the space between these layers to manufacture a dye-sensitized solar cell.

When artificial sunlight (1,000 $W/m^2$) was applied to this dye-sensitized solar cell from the glass substrate side having a titanium oxide layer by using the XC-100A artificial sunlight illuminating lamp (of Violet Co., Ltd.) to measure the photoelectric conversion efficiency, it was 5.6 %. Thereafter, the application of artificial sunlight was continued to measure the time elapsed until the value of this conversion efficiency became 1/2 of the initial value right after the start of activation as half-value period. It was 967 hours.

Comparative Example 2

When a dye-sensitized solar cell was manufactured and evaluated in the same manner as in Example 5 except that the N3 dye was used, the conversion efficiency was 4.8 % and the half-value period was 767 hours.

Example 6

Synthesis of ligand

40 g of 4,4' -dimethyl-2,2'-bipyridine was added little by little to 1 liter of 98 wt% concentrated sulfuric acid under stirring and dissolved in the concentrated sulfuric acid. 55 g of potassium dichromate was added to this solution little by little while the temperature of the solution was maintained at 65°C or lower. The reaction mixture was left to be cooled to room temperature (23°C) and poured into 12 liters of iced water under stirring. After 2 hours of agitation, the precipitate was collected by filtration and rinsed with water. The obtained solid was re-dissolved in ether and let pass through a silica gel column to be purified, and the solvent was removed to obtain 3.8 g of a product. It was found that the product was 4-carboxy-4'-methyl-2,2'-bipyridine by $^1$H-NMR analysis.

Synthesis of dye

1.42 mmol of 4-carboxy-4'-methyl-2,2'-bipyridine synthesized above was added to a solution prepared by dissolving 0.71 mmol of Ru(dimethylsulfoxide)$_4$Cl$_2$ in 30 ml of N,N-dimethylformamide. After the mixture was refluxed for 4 hours, 2.8 g of potassium isothiocyanate was added and refluxed for another 4 hours.

The reaction mixture was left to be cooled to room temperature (23°C), N,N-dimethylformamide was removed under reduced pressure, and 600 ml of water was added. Diluted nitric acid was added to adjust the pH of the obtained solution to 2.5 under stirring at room temperature, and the precipitate was collected by filtration. This solid was re-dissolved in methanol and let pass through the Sephadex LH-20 column (product commercially available from Amersham Biosciences Co., Ltd.) to be purified so as to obtain 0.34 g of a product. It was found that the product was represented by the following formula (22) by $^1$H-NMR analysis. This product was designated as S1.

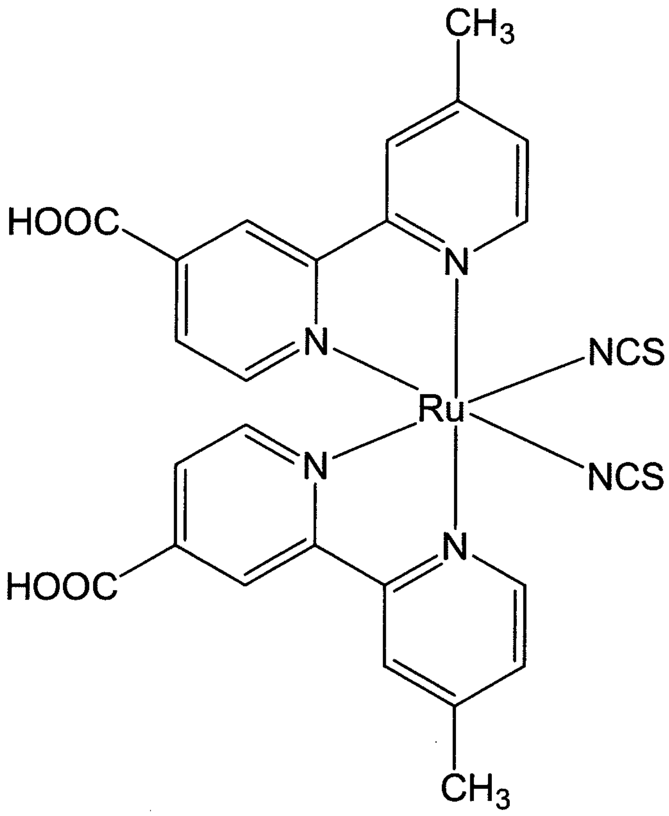

... (22)

Manufacture of dye-sensitized solar cell

12 g of titanium oxide fine particles and 0.2 g of Triton X-100 as a dispersant (product commercially available from Aldrich Co., Ltd.) were added to a mixed solvent of 0.4 ml of acetylacetone and 20 ml of ion exchange water to prepare a dispersion. This dispersion was applied to a 1 mm-thick conducive glass substrate (made of tin oxide, resistance value = 10 Ω/cm$^2$) and heated at 500°C in the air for 1 hour to obtain a conductive glass substrate having a titanium oxide thin film on the surface. This glass substrate was immersed in an ethanol solution containing the dye S1 synthesized above in a concentration of 0.2 mmol/l at room temperature for 24 hours to manufacture a cathode having an oxide thin film electrode which chemically adsorbed the dye of the present invention on the transparent conductive glass.

Meanwhile, platinum was deposited on another conductive glass substrate (thickness of 1 mm, made of tin oxide, resistance value = 10 Ω/cm$^2$) to manufacture an anode.

Further, an electrolyte solution containing 0.1 mol/l of iodine and 0.5 mol/l of lithium iodide dissolved in acetonitrile was prepared.

The above cathode and the anode were opposed to each other, and the above electrolyte solution was held between them to manufacture a dye-sensitized solar cell.

Evaluation of dye-sensitized solar cell

When artificial sunlight was applied to the above manufactured dye-sensitized solar cell at an illuminance of 1,000 W/m$^2$ by using the WXS-50S-1.5 solar simulator (of WACOM Co., Ltd.) and the initial photoelectric conversion efficiency was measured, it was 6.7 %. Thereafter, when the application of artificial sunlight was continued and the time elapsed until the photoelectric conversion efficiency became half of the initial value was measured as half-value period, it was 1,190 hours.

Example 7

Synthesis of ligand

100 ml of tetrahydrofuran was added to 11 ml of a tetrahydrofuran solution of lithium diisopropylamide having

a concentration of 2 mol/l and cooled to -70°C. 2.0 g of 4-carboxy-4'-methyl-2,2'-bipyridine powders synthesized in the step of "synthesis of ligand" in Example 1 was gradually added to the solution while the solution was stirred at -70°C. Thereafter, stirring was continued at -10°C for 1 hour, and then 100 ml of a tetrahydrofuran solution containing 5.5 g of dodecyl bromide was added dropwise at -10°C. The reaction mixture was heated up to room temperature and further stirred for 1 hour.

Thereafter, 250 ml of iced water was added, and the pH of the reaction solution was adjusted to 2.0 with concentrated hydrochloric acid. A water layer was extracted with ether, concentrated, dried and let pass through a silica gel column to remove the solvent to be purified so as to obtain 1.1 g of a product. It was found that the product was 4-carboxy-4'-tridecyl-2,2'-bipyridine by $^1$H-NMR analysis.

Synthesis of dye

0.42 g of a product was obtained substantially in the same manner as in Example 6 except that 1.42 mmol of 4-carboxy-4'-tridecyl-2,2'-bipyridine synthesized above was used in place of 1.42 mmol of 4-carboxy-4'-methyl-2,2'-bipyridine used in the "synthesis of dye" in Example 6. It was found that the product was one represented by the following formula (23) by $^1$H-NMR analysis. This product was designated as S2.

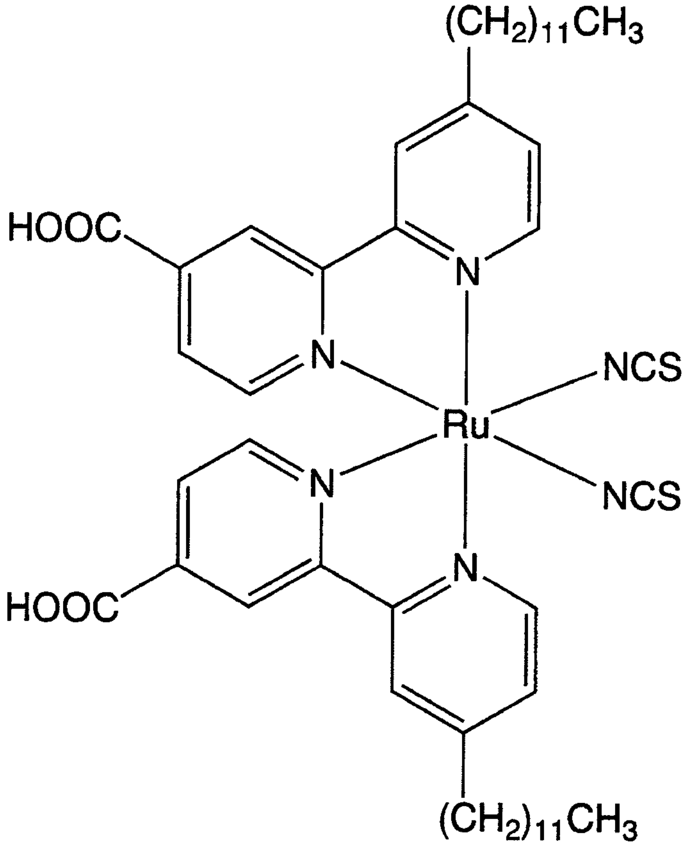

··· (2 3)

Manufacture and evaluation of dye-sensitized solar cell

A dye-sensitized solar cell was manufactured substantially in the same manner as in Example 6 except that the dye S2 was used in place of the dye S1 used in the "manufacture of dye-sensitized solar cell" in Example 6 and evaluated in the same manner as the "evaluation of dye-sensitized solar cell" in Example 6. The results are shown in Table 2.

Example 8

Synthesis of ligand

30 g of 4,4'-dicarboxy-2,2'-bipyridine and 500 g of thionyl chloride were refluxed for 3 hours. After unreacted thionyl chloride was removed, 500 ml of methylene chloride, 17 g of diethylamine and 1.5 g of 4-dimethylaminopyridine were added and stirred at room temperature for 24 hours. Thereafter, the reaction mixture was washed with diluted hydrochloric acid and purified by a silica gel column to remove the solvent so as to obtain 5.6 g of a product. It was found that the product was 4-carboxy-4'-N,N-diethylaminocarbonyl-2,2'-bipyridine by $^1$H-NMR analysis.

Synthesis of dye

0.31 g of a product was obtained substantially in the same manner as in Example 6 except that 1.42 mmol of 4-carboxy-4'-N,N-diethylaminocarbonyl-2,2'-bipyridine synthesized above was used in place of 1.42 mmol of 4-carboxy-4'-methyl-2,2'-bipyridine used in the "synthesis of dye" in Example 6. It was found that the product was one represented by the following formula (24) by $^1$H-NMR analysis. This product was designated as S3.

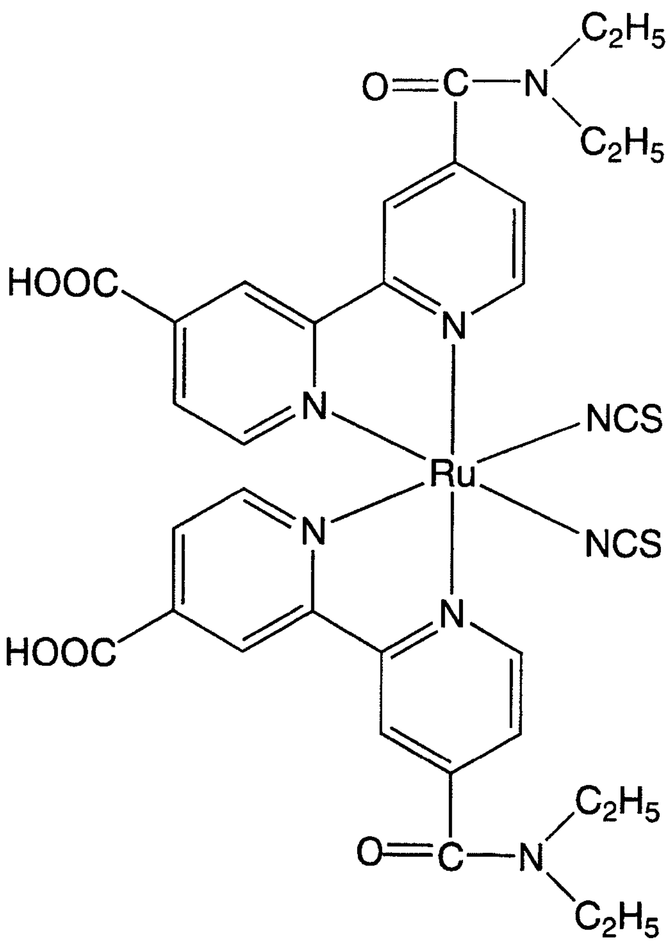

··· (2 4)

Manufacture and evaluation of dye-sensitized solar cell

A dye-sensitized solar cell was manufactured substantially in the same manner as in Example 6 except that the dye S3 was used in place of the dye S1 used in the "manufacture of dye-sensitized solar cell" in Example 6 and evaluated in the same manner as the "evaluation of dye-sensitized solar cell" in Example 6. The results are shown in Table 2.

Example 9

Synthesis of ligand

3.2 g of a product was obtained substantially in the same manner as in the "synthesis of ligand" in Example 8 except that 46 g of N-methyldodecylamine was used in place of 17 g of diethylamine used in the "synthesis of ligand" in Example 8. It was found that the product was 4-carboxy-4'-N-methyl-N-dodecylaminocarbonyl-2,2'-bipyridine by $^1H$-NMR analysis.

Synthesis of dye

0.39 g of a product was obtained substantially in the same manner as in Example 5 except that 1.42 mmol of 4-carboxy-4'-N-methyl-N-dodecylaminocarbonyl-2,2'-bipyridine synthesized above was used in place of 1.42 mmol of 4-carboxy-4'-methyl-2,2'-bipyridine used in the "synthesis of dye" in Example 6. It was found that the product was one represented by the following formula (25) by $^1H$-NMR analysis. This product was designated as S4.

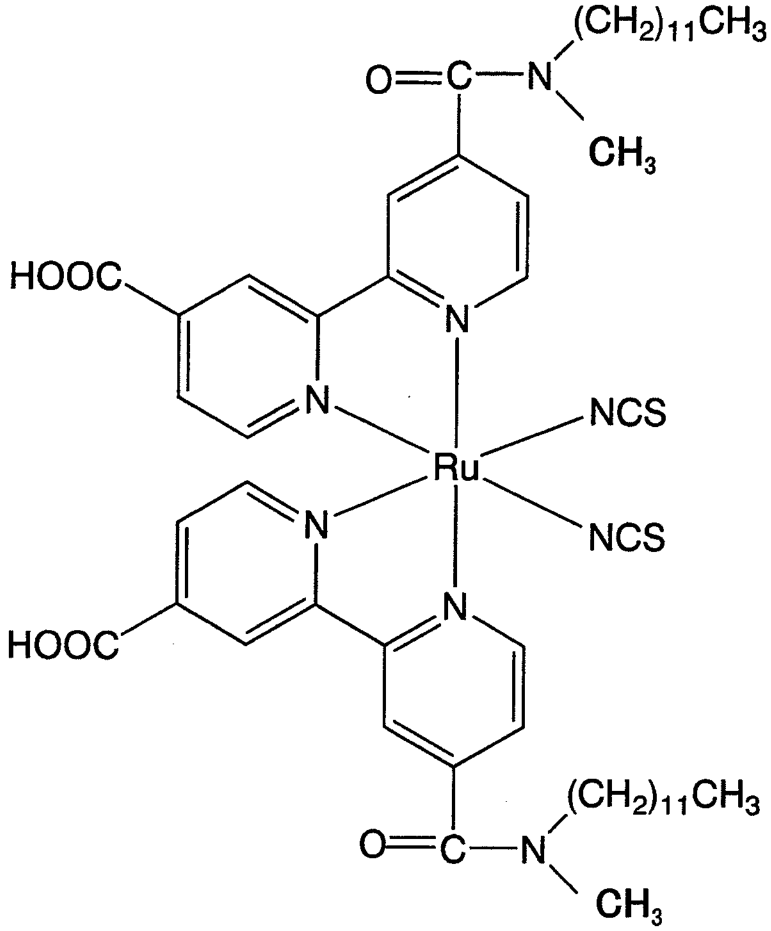

··· (25)

Manufacture and evaluation of dye-sensitized solar cell

A dye-sensitized solar cell was manufactured substantially in the same manner as in Example 6 except that the dye S4 was used in place of the dye S1 used in the "manufacture of dye-sensitized solar cell" in Example 6 and evaluated in the same manner as the "evaluation of dye-sensitized solar cell" in Example 6. The results are shown in Table 2.

Table 2

| | Dye | Photoelectric conversion efficiency (%) | Half-value (hours) |
|---|---|---|---|
| Ex. 6 | S1 | 6.7 | 1190 |
| Ex. 7 | S2 | 7.4 | 1940 |
| Ex. 8 | S3 | 6.5 | 1090 |
| Ex. 9 | S4 | 7.5 | 1870 |

Example 10

Synthesis of dye

57 mg of a product was obtained in the same manner as the "synthesis of dye" in Example 5 except that 34.28 mg of 4-carboxy-4'-methyl-2,2'-bipyridine (synthesized in the "synthesis of ligand" in Example 5) was used in place of 39. 08 mg of 4,4' -dicarboxy-2,2' -bipyridine used in the "synthesis of dye" in Example 5. It was found that the product was one represented by the above formula (22) by $^1$H-NMR analysis.
$^1$H-NMR (DMSO-d6, 298K, 270MHz, $\delta$(ppm)) ; $\delta$ = 9.41 (m, 1H), 9.06-8.70 (m, 5H), 8.27 (m, 1H), 7.82-7.12 (m, 5H), 2.68 (s, 3H), 2.42 (s, 3H)

manufacture and evaluation of dye-sensitized solar cell

When the procedure of the "manufacture and evaluation of dye-sensitized solar cell" in Example 5 was repeated except that the dye synthesized above was used, the conversion efficiency was 5.3 % and the half-value period was 945 hours.

## Claims

1. A dye represented by the following formula (1):

$$ML^1L^2X^1X^2 \qquad (1)$$

wherein M is an element of any one of the groups 8 to 10 of the long form of the periodic table, $L^1$ and $L^2$ are each independently either one of bidentate ligands represented by the following formulas (2) and (3), and $X^1$ and $X^2$ are each independently a monovalent atomic group or unidentate ligand,

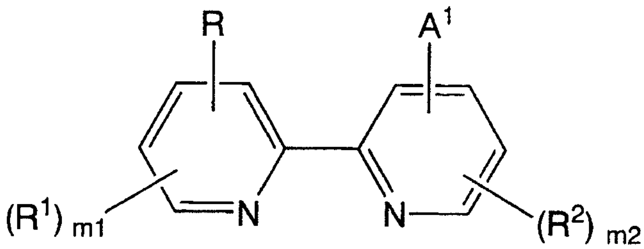

... (2)

wherein $A^1$ is a carboxyl group, sulfonic acid group, phosphoric acid group or group corresponding to a salt thereof, R, $R^1$ and $R^2$ are each independently a monovalent organic group, and m1 and m2 are each independently an integer of 0 to 3.

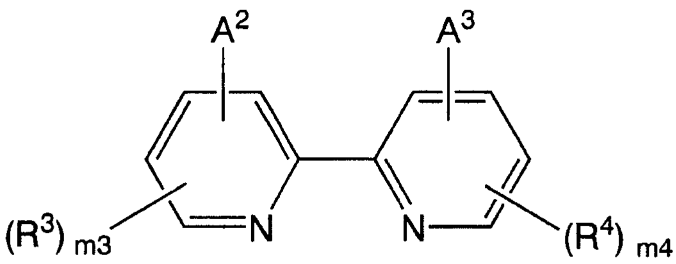

... (3)

wherein $A^2$ and $A^3$ are each independently a carboxyl group, sulfonic acid group, phosphoric acid group or group corresponding to a salt thereof, $R^3$ and $R^4$ are each independently a monovalent organic group, and m3 and m4 are each independently an integer of 0 to 3, with the proviso that when both $L^1$ and $L^2$ are bidentate ligands represented by the formula (3), both $A^2$ and $A^3$ are not carboxyl groups or groups corresponding to a salt thereof.

**2.** The dye according to claim 1, wherein both $L^1$ and $L^2$ are bidentate ligands represented by the above formula (2) in the above formula (1).

**3.** The dye according to claim 1, wherein M is ruthenium in the above formula (1).

**4.** The dye according to claim 1, wherein R is an alkyl group having 1 to 50 carbon atoms or an alkylaminocarbonyl group having 3 to 50 carbon atoms in the above formula (2).

**5.** The dye according to any one of claims 1 to 4 which is used for dye-sensitized solar cells.

**6.** A dye-sensitized solar cell comprising the dye of any one of claims 1 to 4.

## INTERNATIONAL SEARCH REPORT

International application No.
PCT/JP2005/007486

A. CLASSIFICATION OF SUBJECT MATTER
Int.Cl[7] C09B57/10, C07D213/79, H01L31/04, H01M14/00//C07F15/00, C09K3/00

According to International Patent Classification (IPC) or to both national classification and IPC

B. FIELDS SEARCHED

Minimum documentation searched (classification system followed by classification symbols)
Int.Cl[7] C09B57/10, C07D213/79, H01L31/04, H01M14/00//C07F15/00, C09K3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2001-291534 A (Fuji Photo Film Co., Ltd.), 19 October, 2001 (19.10.01), Claims; pages 47, 62 to 63 (Family: none) | 1-3,5,6<br>4 |
| X<br>A | JP 10-504521 A (ECOLE POLYTECHNIQUE FEDERALE DE LAUSANNE), 06 May, 1998 (06.05.98), Claims; page 22, lines 16 to 27 & WO 95/29924 A1 & EP 758337 A1 & US 5789592 A | 1,3,5,6<br>2,4 |
| X<br>A | JP 2001-60467 A (Fuji Photo Film Co., Ltd.), 06 March, 2001 (06.03.01), Claims; pages 13 to 17, 20, 26 & EP 1049117 A1 & JP 2000-311723 A & JP 2001-59062 A | 1,3,5,6<br>2,4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>04 July, 2005 (04.07.05) | Date of mailing of the international search report<br>19 July, 2005 (19.07.05) |
| Name and mailing address of the ISA/<br>Japanese Patent Office<br>Facsimile No. | Authorized officer<br><br>Telephone No. |

Form PCT/ISA/210 (second sheet) (January 2004)

INTERNATIONAL SEARCH REPORT

International application No.
PCT/JP2005/007486

C (Continuation). DOCUMENTS CONSIDERED TO BE RELEVANT

| Category* | Citation of document, with indication, where appropriate, of the relevant passages | Relevant to claim No. |
|---|---|---|
| A | JP 7-500630 A (ECOLE POLYTECHNIQUE FEDERALE DE LAUSANNE),<br>19 January, 1995 (19.01.95),<br>& WO 94/4497 A1 & EP 613466 A1<br>& US 5463057 A | 1-6 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4927721 A
- WO 9850393 A


### Non-patent literature cited in the description


- *J. Am. Chem. Soc.,* 1993, vol. 115, 6382-6390
- *J. Am. Chem. Soc.,* 2001, vol. 123, 1613-1624